## Europäisches Patentamt

## European Patent Office

## Office européen des brevets

(11) Veröffentlichungsnummer: **0 175 969**
**A1**

(12) # EUROPÄISCHE PATENTANMELDUNG

(21) Anmeldenummer: **85111144.3**

(22) Anmeldetag: **04.09.85**

(51) Int. Cl.⁴: **C 07 D 333/08**

(30) Priorität: **13.09.84 DE 3433814**

(43) Veröffentlichungstag der Anmeldung:
**02.04.86 Patentblatt 86/14**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Eichler, Klaus, Dr.**
**Im Traminer 10**
**D-6236 Eschborn 2(DE)**

(72) Erfinder: **Leupold, Ernst Ingo, Dr.**
**Am Zäunefeld 15**
**D-6392 Neu-Anspach(DE)**

(54) **Verfahren zur Herstellung einfach alkylsubstituierter Thiophene.**

(57) Die Erfindung betrifft ein Verfahren zur Herstellung von Alkylthiophenen aus Thiophen und Alkoholen an Zeolith-Katalysatoren vom Pentasil-Typ, wobei H-ZSM-5 bevorzugt wird. Insbesondere lassen sich nach diesem Verfahren Gemische aus 2- und 3-Methylthiophen, bzw. 2- und 3-Ethylthiophen mit hohen Anteil an den 3-Isomeren herstellen.

EP 0 175 969 A1

Croydon Printing Company Ltd

HOECHST AKTIENGESELLSCHAFT    HOE 84/F 213    Dr.MA/cr

<u>Verfahren zur Herstellung einfach alkylsubstituierter
Thiophene</u>

Die vorliegende Erfindung betrifft ein Verfahren zur Herstellung von einfach alkylsubstituierten Thiophenen, d.h.
2- oder 3-Alkylthiophenen oder deren Gemischen.

Alkylierte Thiophene sind wertvolle Vorprodukte. So dienen zum Beispiel die Methylthiophene als Ausgangsstoffe für die Herstellung von Thenylchlorid und Thiophencarbonsäure, die bei der Herstellung von Pharmazeutika benötigt werden (Ullmanns Enzyklopädie der technischen Chemie, 4. Auflage, Band 23, Seite 219). Sie werden im allgemeinen durch Cyclisierungsreaktionen von Olefinen oder Alkoholen mit $H_2S$ oder $CS_2$ hergestellt (GB-PS 1 345 203, DE-OS 2 757 816). Die Synthese der 3-substituierten Alkylthiophene erfordert dabei verzweigte Ausgangsstoffe, die insbesondere bei größeren Alkylsubstituenten sehr teuer sind oder nicht zur Verfügung stehen.

Es bestand daher die Aufgabe, ein Verfahren zur Herstellung von 2- oder 3-Alkylthiophenen zu entwickeln, das von preiswerten Substanzen ausgeht und mit hohen Ausbeuten arbeitet. Es ist bereits bekannt, daß Alkylthiophene durch Alkylierung von Thiophen mit Alkoholen an Zeolithen vom Typ NdNaX hergestellt werden können (E.A. Karakhanov et al., Chemical Abstracts <u>98</u>, 71853 q), allerdings bilden sich dabei vorwiegend mehrfach alkylierte Produkte und es muß das außerordentlich teure Neodym zur Herstellung des Katalysators eingesetzt werden.

Es wurde nun überraschend gefunden, daß sich Thiophen mit Alkoholen an Zeolith-Katalysatoren vom Pentasil-Typ mit hoher Ausbeute zu Gemischen aus 2- oder 3-Alkylthiophenen umsetzen läßt. Insbesondere ist der hohe Anteil an 3-Alkylthiophenen hervorzuheben, da sich bei der elektrophilen aromatischen Substitution von Thiophen normalerweise fast ausschließlich die 2-Isomeren bilden (Ullmanns Enzyklopädie der technischen Chemie, 4. Auflage, Band 23, Seite 218).

Gegenstand der Erfindung ist daher ein Verfahren zur Herstellung einfach alkylsubstituierter Thiophene durch Umsetzung von Thiophen mit einem geradkettigen oder verzweigten aliphatischen Alkohol mit ein bis sechs C-Atomen, dadurch gekennzeichnet, daß die Alkylierung in der Gasphase an einem Zeolith vom Pentasil-Typ durchgeführt wird.

Insbesondere ist Gegenstand der Erfindung ein Verfahren zur derartigen Herstellung von Gemischen aus 2- und 3-Methylthiophen ausgehend von Thiophen und Methanol, sowie von Gemischen aus 2- und 3-Ethylthiophen ausgehend von Thiophen und Ethanol.

Aufgrund des Standes der Technik war es überraschend und in keiner Weise vorhersehbar, daß sich Alkylthiophene in so einfacher Weise und mit derart hoher Ausbeute, wie die Beispiele zeigen, aus Thiophen und Alkoholen darstellen lassen, da es bekannt ist, daß sich Alkohole an derartigen Zeolith-Katalysatoren zu Olefinen und Aromaten umsetzen. Insbesondere überrascht dabei der hohe Anteil an den sonst in vielen Fällen schwer zugänglichen 3-Alkylthiophenen. Der Vergleich von Beispiel 1 und 2 zeigt, daß durch einen Überschuß von Thiophen der Dialkylthiophen-Anteil deutlich zurückgedrängt werden kann. Aus den Beispielen 2 und 3 geht ferner hervor, daß die Ausbeute an Alkylthiophenen bezogen auf den eingesetzten Alkohol fast vollständig ist.

Zur Durchführung des erfindungsgemäßen Verfahrens werden Thiophen und ein geradkettiger oder verzweigter aliphatischer Alkohol mit ein bis sechs C-Atomen verdampft und über den Zeolith-Katalysator vom Pentasil-Typ geleitet.

Für den Begriff Pentasile gilt dabei die Definition von Kokotailo und Meier ("Pentasil family of high silicon crystalline materials" in Special Publication No. 33 of the Chemical Society, London 1980). Die Pentasil-Familie umfaßt beispielsweise die synthetischen Zeolithe ZSM-5 (US-PS 3 702 886), ZSM-8 (GB-PS 1 334 243), ZSM-11 (US-PS 3 709 979) und ZSM-23 (US-PS 4 076 842), wobei ZSM-5 bevorzugt wird.

Das Si/Al-Verhältnis der Pentasile liegt vorzugsweise bei 20 bis 2 000. Pentasile mit einem höheren Aluminiumgehalt lassen sich durch Behandlung mit Mineralsäuren, organischen Säuren oder chelatisierenden Substanzen auf das gewünschte Si/Al-Verhältnis einstellen, wobei ein Teil des Aluminiums aus dem Zeolith-Gitter entfernt wird.

Bei dem erfindungsgemäßen Verfahren werden die Zeolithe vorzugsweise in ihrer sauren Form eingesetzt. Diese sauren Formen lassen sich nach bekannten Methoden aus den Alkalimetall-Formen, wie sie in der Regel bei der Zeolith-Synthese anfallen, durch vollständigen oder partiellen Ionenaustausch herstellen. Eine übliche Methode zur Herstellung der H-Form eines Zeoliths besteht beispielsweise darin, die Alkalimetall-Form zunächst durch partiellen oder vollständigen Ionenaustausch mit einer Ammoniumsalz-Lösung in die Ammoniumform und diese anschließend durch Kalzinieren in die H-Form zu überführen. Aber auch die mit Alkali-, Erdalkali- und mit Seltenerdmetall-Ionen ausgetauschten Formen zeigen katalytische Aktivität.

Für das erfindungsgemäße Verfahren sind auch Zeolithe geeignet, bei denen Aluminium- oder Siliciumatome durch andere Gitteratome wie Bor, Eisen, Gallium, Germanium, Titan oder Zirkon ersetzt sind.

Die erfindungsgemäßen Zeolith-Katalysatoren bestehen im allgemeinen aus der katalytisch aktiven Zeolith-Komponente sowie einem Bindermaterial. Letzteres ist erforderlich, um den Zeolith in eine für das erfindungsgemäße Verfahren geeignete äußere Form zu bringen.

Als Bindermaterial eignen sich vor allem Oxide oder Hydroxide des Aluminiums und die Oxide bzw. Hydroxide des Siliciums, sowie Schichtsilicate, beispielsweise aus der Kaolin- oder Montmorillonit-Familie, sowie auch Titandioxid.

Dieser so hergestellte Zeolith-Katalysator wird gewöhnlich vor dem Einsatz in der erfindungsgemäßen Alkylierungsreaktion zunächst durch Kalzinieren bei Temperaturen zwischen 300 und 700°C aktiviert. Zur besseren Stabilisierung des Katalysators ist es manchmal vorteilhaft, die Kalzinierung in Gegenwart von Wasserdampf, Ammoniak oder deren Mischungen durchzuführen.

Eine günstige einfache Verfahrensweise zur Durchführung der erfindungsgemäßen Alkylierung besteht darin, Thiophen und den zur Alkylierung verwendeten Alkohol einzeln oder als Gemisch aus einer Dosiervorrichtung zuerst in einen Verdampfer zu leiten und das entstandene Gas dann durch ein von außen beheiztes und mit dem Katalysator gefülltes Reaktionsrohr (Festbett-Reaktor) zu leiten. Der Verdampfer kann dabei auch einfach aus einer Verlängerung des Reaktionsrohres, die mit inertem Material zur Vergrößerung der Oberfläche gefüllt ist, bestehen. Das molare Verhältnis von Thiophen zu dem bei der Alkylierung verwendeten Alkohol liegt vorzugsweise im Bereich zwischen 1:2 und 20:1.

Als Alkohole zur Durchführung des erfindungsgemäßen Verfahrens eignen sich Methanol, Ethanol, n-Propanol, iso-Propanol, n-Butanol, tert.-Butanol oder andere geradkettige oder verzweigte Alkohole mit maximal sechs C-Atomen.

In der Verdampfungs- oder Erwärmungszone erfolgt gegebenenfalls noch eine Vermischung mit Wasserstoff, Stickstoff, Wasserdampf, Argon und/oder einem anderen Trägergas. Es hat sich dabei als vorteilhaft erwiesen, diese Gase vor der Vermischung auf Reaktionstemperatur aufzuheizen.

Die erfindungsgemäße Alkylierung ist jedoch nicht auf diese Verfahrensweise (Festbett-Reaktor) beschränkt, sondern läßt sich im Prinzip auch in anderen geeigneten Reaktor-Typen durchführen (z.B. Wirbelschicht-Reaktor). Vorteilhaft ist auch die Verwendung eines Hordenreaktors mit Alkohol-Zwischeneinspeisung, da dadurch an allen Stellen des Reaktors ein hohes Thiophen/Alkohol-Verhältnis gewährleistet ist und kaum Reaktionsprodukte des Alkohols mit sich selbst gebildet werden.

Die erfindungsgemäße Alkylierung wird im allgemeinen bei Temperaturen zwischen 150 und 550°C, vorzugsweise bei 200 bis 450°C, sowie Drücken von 0,1 bis 10 bar, vorzugsweise bei Normaldruck, durchgeführt.

Die Belastung des Zeolith-Katalysators - ausgedrückt als LHSV (Liquid Hourly Space Velocity, $h^{-1}$) - liegt dabei im allgemeinen zwischen 0,05 und 10 $h^{-1}$, vorzugsweise zwischen 0,1 und 5 $h^{-1}$.

Die Reaktionsprodukte werden nach Verlassen des Reaktors zur Abtrennung der kondensierbaren Anteile gekühlt.

Das entstandene Isomerengemisch kann nach bekannten Verfahren destillativ getrennt werden. Das nicht umgesetzte Ausgangsprodukt kann in den Reaktor zurückgeführt werden.

Falls die Aktivität des Katalysators langsam aufgrund einer Verkokung abnimmt, kann er von Zeit zu Zeit regeneriert werden. Dies geschieht, indem Sauerstoff, Luft, Stickstoff/Luft, Sauerstoff/Luft, Sauerstoff/Inertgas oder Luft/Inertgas bei Temperaturen zwischen 300 und 650°C über den desaktivierten Katalysator geleitet wird. Stickstoff/-Luft wird dabei bevorzugt. Die Temperatur sollte dabei an keiner Stelle des Reaktors 650°C übersteigen.

Die Erfindung soll durch die folgenden Beispiele erläutert werden, wobei diese aber in keiner Weise einschränkend sein sollen.

Beispiele
Katalysator-Herstellung

100 g ZSM-5-Pulver in der Na-Form (US-PS 3 702 886, Beispiel 1) wurden bei 100°C dreimal für fünf Stunden mit 1-molarer Ammoniumchloridlösung behandelt, gewaschen, getrocknet und fünf Stunden bei 550°C in Luft calciniert. 65 g des dabei erhaltenen Pulvers wurden mit 35 g $Al_2O_3$ zu Strangpresslingen von 1,6 mm Durchmesser verarbeitet, vier Stunden bei 500°C calciniert, auf eine Teilchengröße von 0,25 bis 1,0 mm zerkleinert und bei 450°C im Stickstoffstrom zwei Stunden lang calciniert.

Beschreibung der Apparatur

15 ml des oben beschriebenen Katalysators wurden in einen Rohrreaktor aus Glas von 16 mm Innendurchmesser und 50 cm Länge eingefüllt und mit Glaskugeln (zum Verdampfen der flüssigen Reaktanden) überschichtet. Der Reaktor befand sich in einem elektrisch beheizten Ofen. Flüssige Reaktanden wurden über eine Dosierpumpe zugeführt, Gase über eine Gasversorgung, bestehend aus Reduzierventilen und Vorrichtungen zum Messen des Drucks und der Durchflußmenge. Die kondensierbaren Reaktionsprodukte wurden in einer Kühlfalle bei 0°C kondensiert und gaschromatographisch analysiert.

Beispiel 1: (Alkylierung von Thiophen mit Methanol)

8 ml/h eines Gemisches aus Thiophen und Methanol im molaren Verhältnis 1 : 1 wurden zusammen mit 5 l/h Stickstoff über den oben beschriebenen Katalysator geleitet. Nach einer kurzen Vorlaufzeit von etwa 15 Minuten zur Einstellung konstanter Betriebsbedingungen wurde alle zwei Stunden die Vorlage gewechselt und anschließend analysiert. Tabelle 1 zeigt die Ergebnisse:

|  |  | Produktgehalt | | |
| Dauer | Temperatur | 2-Methylthiophen | 3-Methylthiophen | Dimethylthiophene |
| (h) | (°C) | (Gew.-%) | (Gew.-%) | (Gew.-%) |
| 2 | 200 | 1,1 | 0,5 | 0,1 |
| 4 | 250 | 1,3 | 0,6 | 0,1 |
| 6 | 300 | 5,8 | 3,0 | 0,9 |
| 8 | 350 | 8,2 | 4,7 | 3,3 |
| 10 | 400 | 11,7 | 7,5 | 12,2 |
| 12 | 450 | 10,2 | 9,9 | 9,4 |

Tabelle 1: Alkylierung von Thiophen mit Methanol (Verhältnis 1:1)

Beispiel 2: (Alkylierung von Thiophen mit Methanol)

8 ml/h eines Gemisches aus Thiophen und Methanol im molaren Verhältnis 5 : 1 wurden zusammen mit 5 l/h Stickstoff über den oben beschriebenen Katalysator geleitet. Nach einer kurzen Vorlaufzeit von etwa 15 Minuten zur Einstellung konstanter Betriebsbedingugnen wurde alle zwei Stunden die Vorlage gewechselt und anschließend analysiert. Tabelle 2 zeigt die Ergebnisse:

| Dauer (h) | Temperatur (°C) | P r o d u k t g e h a l t | | |
| | | 2-Methylthiophen (Gew.-%) | 3-Methylthiophen (Gew.-%) | Dimethylthiophene (Gew.-%) |
|---|---|---|---|---|
| 2 | 300 | 4,7 | 2,4 | 0,8 |
| 4 | 350 | 9,1 | 5,0 | 3,1 |
| 6 | 400 | 7,5 | 7,2 | 3,0 |
| 8 | 450 | 6,2 | 6,1 | 2,3 |
| 12 | 450 | 5,1 | 4,0 | 1,4 |
| 16 | 450 | 6,3 | 2,5 | 1,6 |
| 20 | 450 | 8,3 | 1,8 | 1,9 |
| 26 | 450 | 8,5 | 1,2 | 1,7 |

Tabelle 2: Alkylierung von Thiophen mit Methanol (Verhältnis 5:1)

Beispiel 3 (Alkylierung von Thiophen mit Ethanol)

8 ml/h eines Gemisches aus Thiophen und Ethanol im molaren Verhältnis 5 : 1 wurden zusammen mit 5 l/h Stickstoff über den oben beschriebenen Katalysator geleitet. Nach einer kurzen Vorlaufzeit von etwa 15 Minuten zur Einstellung konstanter Betriebsbedingugnen wurde alle zwei

0175969

Stunden die Vorlage gewechselt und anschließend analysiert. Tabelle 3 zeigt die Ergebnisse:

| Dauer (h) | Temperatur (°C) | Produktgehalt | | |
|---|---|---|---|---|
| | | 2-Ethylthiophen (Gew.-%) | 3-Ethylthiophen (Gew.-%) | Diethylthiophene (Gew.-%) |
| 2 | 300 | 2,8 | 2,3 | 0,6 |
| 4 | 350 | 5,4 | 4,2 | 0,8 |
| 6 | 400 | 9,5 | 7,8 | 1,2 |
| 8 | 450 | 7,5 | 6,4 | 2,9 |

Tabelle 3: Alkylierung von Thiophen mit Ethanol.

0175969
HOE 84/F 213

PATENTANSPRÜCHE:

1. Verfahren zur Herstellung einfach alkylsubstituierter Thiophene durch Umsetzung von Thiophen mit einem geradkettigen oder verzweigten aliphatischen Alkohol mit ein bis sechs C-Atomen, dadurch gekennzeichnet, daß die Alkylierung in der Gasphase an einem Zeolith vom Pentasil-Typ durchgeführt wird.

2. Verfahren nach Anspruch 1, dadurch gekennzeichnet, daß man Methanol einsetzt.

3. Verfahren nach Anspruch 1, dadurch gekennzeichnet, daß man Ethanol einsetzt.

4. Verfahren nach einem der Ansprüche 1 bis 3, dadurch gekennzeichnet, daß der Zeolith Protonen als Kationen enthält.

5. Verfahren nach einem der Ansprüche 1 bis 4, dadurch gekennzeichnet, daß man als Zeolith ZSM-5 einsetzt.

6. Verfahren nach einem der Ansprüche 1 bis 5, dadurch gekennzeichnet, daß man bei Temperaturen zwischen 150 und 550°C arbeitet.

7. Verfahren nach einem der Ansprüche 1 bis 6, dadurch gekennzeichnet, daß man bei einem Druck zwischen 0,1 und 10 bar arbeitet.

8. Verfahren nach einem der Ansprüche 1 bis 7, dadurch gekennzeichnet, daß man in Gegenwart von Wasserstoff, Stickstoff, Wasserdampf, Argon oder einem Gemisch aus diesen arbeitet.

9. Verfahren nach einem der Ansprüche 1 bis 8, dadurch gekennzeichnet, daß man bei einem molaren Verhältnis von Thiophen zu Alkohol von 1 : 2 bis 20:1 arbeitet.

## EINSCHLÄGIGE DOKUMENTE

EP 85111144.3

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | CHEMICAL ABSTRACTS, Band 98, Nr. 9, 28. Februar 1983, Columbus, Ohio, USA<br><br>E.A. KARAKHANON et al. "Alkylation of thiophene by methyl, isopropyl, and tert-butyl alcohols on Nd Na X zeolite"<br>Seite 607, Spalte 2, Zusammenfassung Nr. 71 853q<br><br>& Vestn. Mosk. Univ., Ser.2: Khim. 1982, 23(5), Seiten 495-7<br><br>-- | 1,2,6 | C 07 D 333/08 |
| A | GB - A - 641 944 (TEXACO)<br><br>* Seite 2, Zeilen 36-42,69-81; Ansprüche *<br><br>-- | 1,6,7, 9 | |
| A | G.A.OLAH "Friedel-Crafts and related reactions", Band II, "Alkylation and Related Reactions" Teil 1, 1964<br><br>INTERSCIENCE PUBLISHERS, New York-London-Sidney Seiten 533,534<br><br>* Seite 533, Zeile 28 - Seite 534, Zeile 2 *<br><br>---- | 1,6 | RECHERCHIERTE SACHGEBIETE (Int. Cl.4)<br><br>C 07 D 333/00 |

Der vorliegende Recherchenbericht wurde für alle Patentansprüche erstellt.

| Recherchenort | Abschlußdatum der Recherche | Prüfer |
|---|---|---|
| WIEN | 10-12-1985 | HOFBAUER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82